# EUROPÄISCHE PATENTSCHRIFT

(11) **EP 0 366 945 B1**
(45) Veröffentlichungstag und Bekanntmachung des Hinweises auf die Patenterteilung: **16.06.1993**
(21) Anmeldenummer: 89118184.4
(22) Anmeldetag: 30.09.1989
(51) Int. Cl.: A61F 2/36

(54) **Femurprothese mit einem Satz Rippenteile**
Femoral prosthesis with a set of fins
Prothèse fémorale avec un jeu d'ailettes

(30) Priorität: 04.10.1988 CH 3691/88
(43) Veröffentlichungstag der Anmeldung: 09.05.1990
(73) Patentinhaber: Intraplant AG, CH-6330 Cham (CH)
(72) Erfinder: Imhof,Martin, 6343 Rotkreuz (CH)
(74) Vertreter: Patentanwälte Schaad, Balass, Menzl & Partner AG

(56) Entgegenhaltungen:
- EP-A- 0 131 178
- EP-A- 0 149 527
- EP-A- 0 170 982
- EP-A- 0 201 442
- EP-A- 0 234 358
- EP-A- 0 244 610
- EP-A- 0 266 081
- WO-A-84/03037
- WO-A-88/01492
- FR-A- 1 278 359
- GB-A- 2 159 416
- US-A- 3 918 441

## Beschreibung

Die Erfindung betrifft eine Femurprothese nach dem Oberbegriff des Anspruches 1.

Wie beispielsweise aus der WO-A-8 403 037 bekannte Prothesen dieser Art werden z. B. im Diaphysenbereich mit dem distalen Ende des Schaftblatts verklemmt oder verkeilt und im proximalen Bereich des bearbeiteten Femurs verankert. Nachstehend wird auf die Verankerung im proximalen Bereich Bezug genommen.

Für die Verankerung besitzen die bekannten Prothesen der eingangs genannten Art auf jeder Seite des Schaftblattes eine den gesamten halsnahen Bereich einnehmende, querverlaufende Nut. In diese Nuten wird bei der Implantation zur Anpassung der Prothesendicke je ein flächiges, die ganze Breite des Schaftblatts überdeckendes und im wesentlichen die Höhe des Trochanterflügels besitzendes Rippenteil eingesetzt. Die Rippenteile besitzen in Längsrichtung des Schaftblatts verlaufende Rippen, um eine für nachwachsendes Knochengewebe geeignete Verankerung der Prothese zu ermöglichen.

Bei diesen Prothesen wird als nachteilig empfunden, dass trotz der im Satz mit verschiedener Dicke zur Verfügung stehenden Rippenteile eine einwandfreie Passung der Prothese in der Implantationshöhlung nur sehr schwer erreicht werden kann:
Sobald nämlich die Implantationshöhlung dem Prothesenquerschnitt entsprechend vorbereitet ist, erfolgt eine Feinbearbeitung der Höhlungswände im Hinblick auf die Oberflächenform der einzusetzenden Rippenteile. Gelingt die Feinbearbeitung nicht vollständig, z. B. bezüglich der Neigung der ausmodellierten Höhlungswand oder durch lokal überhöhten Abtrag, kann die Prothese im Femur nicht einwandfrei eingepasst werden und trägt nur teilweise. In der Folge können lokale Ueberbeanspruchungen oder längerfristig eine Verlagerung der implantierten Prothese entstehen.

Solch eine mangelhafte Passung ist zwar in der Regel durch eine Nachbearbeitung des Femurendes unter Verwendung eines nächstdickeren Rippenelementes korrigierbar. Dann stellt sich aber wieder dasselbe handwerkliche Problem; zudem ist besonders im Trochanterbereich wegen des Ansatzes von Muskeln und Bändern das Abtragen weiterer Knochensubstanz unerwünscht.

Entsprechend ist es die Aufgabe der vorliegenden Erfindung, eine Prothese zu schaffen, bei welcher eine schwierige und zeitraubende Anpassung der Implantationshöhlung entfällt und gleichzeitg einwandfreie Passung der Prothese durch vollständige Anlage aller Rippen an die Wände der Höhlung gewährleistet ist. Insbesondere soll die einmal gewählte Höhlung auch bei Korrekturen im wesentlichen erhalten bleiben.

Zur Lösung dieser Aufgabe besitzt die erfindungsgemässe Femurprothese die kennzeichnenden Merkmale von Anspruch 1.

Durch diese Anordnung wird erreicht, dass in einem Arbeitsgang von der dorsalen bzw. ventralen Höhlungswand nur noch ein lokaler, für die Feinbearbeitung günstig verlaufender Bereich gegengleich zum entsprechenden Prothesenteil modelliert werden muss:
Ausgehend von der nach dem Prothesenquerschnitt vorbereiteten Höhlung mit unregelmässig verlaufenden Wandbereichen können die einzelnen Rippenteile zum Einsetzen in die Prothese ausgewählt werden. Die notwendige Anpassung der Höhlenwand kann dann lokal, Rippenteil für Rippenteil, der jeweiligen Rippe entlang, vorgenommen werden.

Da ein Abmessen einer fertig zu modellierenden Wandfläche in die Tiefe und hinsichtlich Längsneigung weniger Schwierigkeiten bietet als bezüglich Breite und Querneigung, ist dies verhältnismässig einfach. Sollte trotzdem einmal zu viel Knochenmaterial abgetragen werden, kann das Rippenteil unter entspechender Nachbearbeitung gegen ein nächsthöheres ausgetauscht werden. Die Nachbearbeitung erfolgt dann wieder lokal, es ist nicht mehr notwendig, zur Korrektur die gesamte dorsale oder ventrale Höhlungswand abzutragen; der weitere Verlust an Knochenmatial hält sich in vertretbaren Grenzen.

Bevorzugte Ausführungsformen weisen Merkmale der abhängigen Ansprüche auf.

Nachstehend sind Ausführungsbeispiele der vorgeschlagenen Femurprothese anhand der Figuren näher beschrieben. Es zeigt:
- Fig. 1a: eine Ansicht der dorsalen bzw. ventralen Seite einer erfindungsgemässen Prothese mit einem zugehörigen Rippenteil;
- Fig. 1b: eine laterale Ansicht der Prothese der Fig. 1 mit einem weiteren Rippenteil;
- Fig. 2: einen Schnitt durch den halsnahen Bereich der Prothese von Fig. 1 entlang der dort eingezeichneten Linie I-I mit Rippenteilen und diese verriegelnden Mitteln;
- Fig. 3: in auseinandergezogener Darstellung eine weitere Ausführungsform;
- Fig. 4: einen Schnitt längs der Linie IV-IV der Fig. 3 mit eingesetzten Rippenteilen; und
- Fig. 5: in Seiten- bzw. Frontansicht ein Rippenteil für die Prothese gemäss Fig. 3.

In den Figuren sind für sich funktionell entsprechende Bestandteile dieselben Bezugsziffern verwendet.

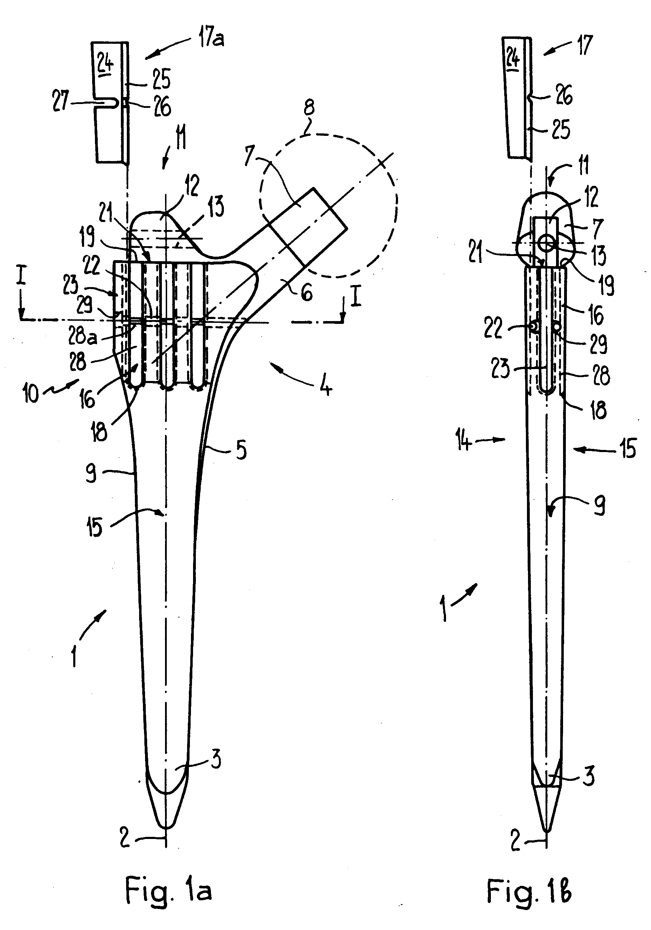

Die Figuren 1a und 1b zeigen das Schaftblatt 1, welches eine Längsachse 2 besitzt und sich von seinem distalen Ende 3 über seine Länge zu einem halsnahen Bereich 4 erweitert. Die mediale Schmalseite 5 des Schaftblattes 1 geht in einen Hals 6 über, welcher in einem Konuszapfen 7 endet, der zur Aufnahme einer Gelenkkugel 8 dient. Die laterale Seite 9 des Schaftblattes 1 erweitert sich zum Trochanterflügel 10; er bildet im wesentlichen die laterale Längsseite des halsnahen Bereichs 4. Am proximalen Ende 11 der Prothese ist ein Höcker 12 mit einer Querbohrung 13 vorgesehen, an welcher der Haken eines Ausschlaginstruments angesetzt werden kann.

Die eine Prothesenseite 14 ist deckungsgleich zur anderen Seite 15 ausgestaltet, so dass die Prothese wahlweise im rechten oder linken Femur eingesetzt werden kann.

Im halsnahen Bereich 4 des Schaftblattes 1 sind mehrere Nuten 16 vorhanden, in welche Rippenteile 17 einsetzbar sind. Die Nuten erstrecken sich parallel zur Längsachse 2 des Schaftblattes 1; ihr distales Ende 18 befindet sich auf der Höhe des distalen Endes des Trochanterflügels 10. Ihr proximales Ende 19 mündet offen in eine Schulter 21, mit welcher der Höcker 12 vom halsnahen Bereich 4 abgesetzt ist. Quer zu den Nuten 16 verläuft eine weiter unten noch näher beschriebene Bohrung 22, welche der Verriegelung der Rippenelemente 17 dient.

Eine weitere, zur Längsachse 2 parallele Nut 23 ist im Trochanterflügel 10 vorgesehen und besitzt dieselbe Konfiguration, insbesondere ein in die Schulter 21 mündendes offenses Ende 19, wie die Nuten 16.

Zur Prothese gehört weiter ein Satz der Rippenteile 17, 17a zum Einsetzen in die Nuten 16, 23. Die Rippenteile 17, 17a liegen im Satz mit unterschiedlich hohen Rippen 24, aber jeweils gleich ausgebildeten, eine Kerbe 26 aufweisenden Fuss 25 vor. Die Rippenteile 17 sind zum Einsetzen in die Nuten 16 bestimmt, während die Rippenteile 17a in der Rippe 24 eine querverlaufende Kerbe 27 aufweisen und damit zum Einsetzen in die Nut 23 vorgesehen sind.

Die Kerben 26, 27 dienen der Arretierung der in die Nuten eingesetzten Rippenteile; sie werden weiter unten zusammen mit der Bohrung 22 näher beschrieben.

Die Rippenfüsse 25 besitzen ein zu den Nuten 16, 23 gegengleiches Schwalbenschwanzprofil, so dass vom offenen Ende 19 her in die Nuten 16, 23 eingeschobene und verriegelte Rippenteile unverrückbar festsitzen.

Für die Implantation wird die Prothese mit dem Schaftblatt 1 soweit als möglich in die Endlage gebracht, derart, dass für die Einpassung des halsnahen Bereichs 4 in der vorbereiteten Implantationshöhlung im Femurende die Rippenteile 17, 17a aus dem Satz ausgewählt werden können.

Danach erfolgt die Modellierung der Höhlungswand Rippenteil für Rippenteil, bis die Prothese durch vollständige Anlage aller Rippenteile an die Höhlungswand einwandfrei eingepasst ist.

Für gewisse Feinarbeiten ist es notwendig, ein Rippenteil aus seiner Nut zu entfernen, damit neben der in der Endlage liegenden Prothese genügend Platz für das die Höhlungswand bearbeitende Werkzeug vorhanden ist. Da das Rippenteil einfach aus dem offenen Ende 19 der Nut 16, 23 herausgezogen werden kann, ist eine Verschiebung der Prothese nicht notwendig. Dies ist vorteilhaft, weil die verbleibende Prothese z. B. durch die Nutenränder eine Orientierungshilfe gegenüber der Höhlungswand bietet, was das präzise Arbeiten wesentlich erleichtert. Weiter ist das mehrmalige Entfernen der Prothese aus ihrer Endlage umständlich und unangenehm, weil sie sich unter Umständen nicht in genau die gleiche Lage zurückschieben lässt und damit das Fertigmodellieren der Höhlungswand erschwert.

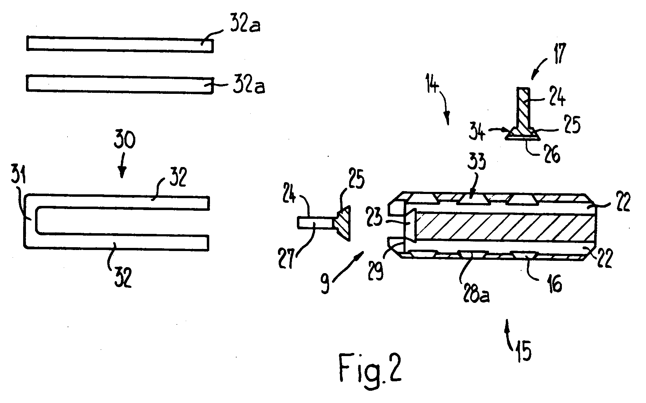

Sobald die Prothese einwandfrei eingepasst ist, werden die Rippenteile 17, 17a verriegelt. Fig. 2 zeigt die entsprechende Anordnung mit Hilfe eines Querschnitts durch den halsnahen Bereich 4 entlang der Linie I-I von Fig. 1a. Je eine Bohrung 22 verläuft quer an der Seite 15 bzw. an der Seite 14, den Nutengrund 28 der Nuten 16 schneidend. Dadurch entsteht im Nutengrund 28 eine Kerbe 28a. An der lateralen Seite 9 münden die Bohrungen 22 in eine Rille 29, welche die Nut 23 quert. Diese Anordnung ist zur Aufnahme eines Bügels 30 bestimmt welcher in Verriegelungslage mit seinem Steg 31 in der Kerbe 27 des Rippenteils 17a aufliegt, mit seinen Schenkeln 32 dagegen durch die Bohrungen 22 ragt und den Nutengrund 28 in den Kerben 28a durchquert.

Nachdem die Prothese in die Höhlung eingepasst ist, wird sie etwas herausgehoben und die Rippenelemente 17, 17a durch Einsetzen des Bügels 30 verriegelt. Nach Wiedereinsetzen der Prothese in ihre Endlage ist auch die Lage des Bügels 30 fixiert, da die an der lateralen Prothesenseite 9 anliegende Knochenwand ein Herausgleiten des Bügels 30 aus seiner Verriegelungslage verunmöglicht.

Abweichend von der in den Figuren gezeigten Ausführungsform ist es für gewisse Anwendungen vorteilhaft, die Nut 23 im Trochanterflügel 10 zur Längsachse 2 des Schaftblattes 1 geneigt anzuordnen. Weiter können auch die Rippenteile 17 eine Kerbe 27 aufweisen, so dass jedes Rippenteil des Satzes wahlweise in eine Nut 16 oder eine Nut 23 einsetzbar ist.

Im Gegensatz dazu kann die Nut 23 eine andere Breite als die Nuten 16 aufweisen, damit die Rippenteile 17, 17a nicht verwechselt werden können.

Weiter ist es denkbar, die Profile von Nuten und Rippenfuss anders als schwalbenschwanzförmig auszugestalten. Es ist jedoch vorteilhaft, dass das Nutenprofil sich gegen das Protheseninnere erweitert und das der Rippenfuss ein gegengleiches Profil aufweist, damit das Rippenteil nach Verriegelung in der Nut gefangen ist.

Wird auf ein Rippenteil 17a in der Nut 23 verzichtet, so können anstelle des Bügels 30 zur Verriegelung der Rippenelemente 17 Stifte 32a verwendet werden, welche in die jeweilige Bohrung 22 einschiebbar sind.

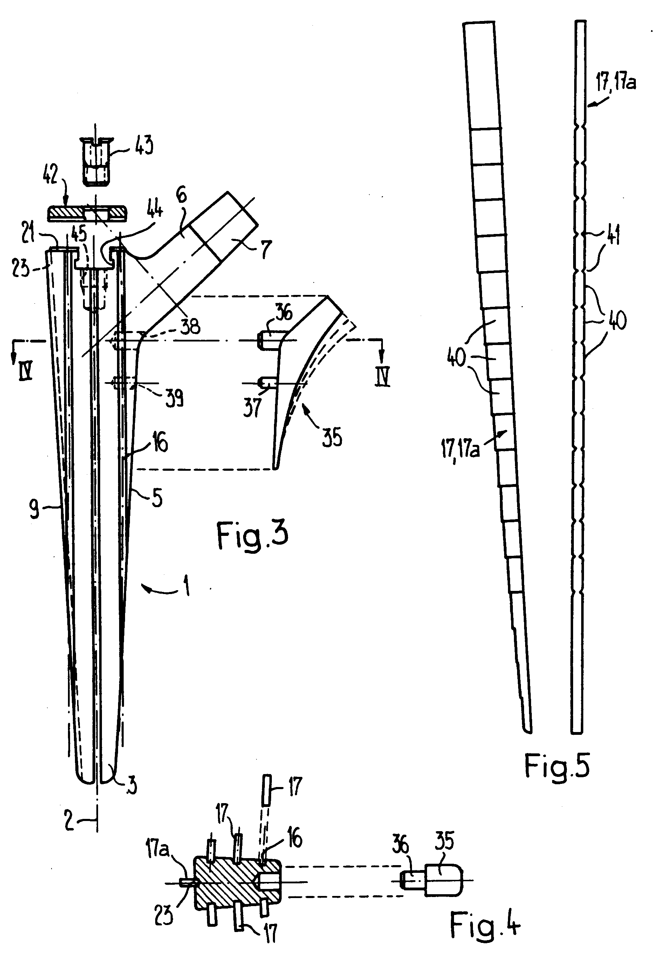

Bei der in den Fig. 3-5 dargestellten Ausführungsform erkennt man, dass die Nuten 16 sich praktisch über die gesamte Länge des Schaftblattes 1 erstrecken und ein rechteckiges Profil (Fig. 4) aufweisen. Ebenso erstreckt sich die Nut 23 auf der lateralen Seite 9 über die gesamta Länge des Schaftblattes 1. Ausserdem hat der Uebergangsbereich zwischen der medialen Schmalseite 5 und dem Hals 6 einen verhältnismässig geringen Krümmungsradius. Um aber diese Ausführungsform der Prothese auch individuell der Krümmung des Calcarbogens an der Eingangsseite der Implantationshöhlung anpassen zu können, ist dieser Prothese ein Satz Abstandhalter 35 zugeordnet, die mittels Zapfen 36,37 in entsprechende Bohrungen 38,39 in der medialen Schmalseite 5 einsteckbar sind. In Fig. 3 ist mit gestrichelten Linien angedeutet, dass der Satz Abstandhalter 35 beispielsweise deren drei aufweist.

Die in die Nuten 16 und 23 einzusetzenden Rippenteile 17 bzw. 17a sind gleich ausgebildet und in Fig. 5 dargestellt. Daraus ist ersichtlich, dass diese Rippenteile 17 bzw. 17a zum distalen Ende 3 des Schaftblattes von abnehmender Höhe sind, wobei diese Höhe stufenweise abnimmt. Zwischen benachbarten Abschnitten 40 konstanter Höhe der Rippenteile 17,17a der Fig. 5 sind Kerben 41 vorhanden, die eine Sollbruchstelle bilden.

Die Implantation der Prothese gemäss Fig. 3 bis 5 wird wie folgt vorgenommen:
In der vorbereiteten Implantationshöhlung wird die Krümmung des Calcarbogens abgeschätzt und es wird der passende Abstandshalter 35 in die mediale Schmalseite 5 des Schaftblattes 1 eingesteckt. Sodann wird die Prothese (noch ohne Rippenteile) in die Implantationshöhlung eingeführt. Nun werden in die Nuten 16 bzw. 23 Rippenteile 17 bzw. 17a eingeschlagen, bis der Chirurg merkt, dass der nach aussen abstehende Scheitelbereich der Rippenteile 17 bzw. 17a an der Innenwand der Implantationshöhlung ansteht. Sodann wird der nach oben noch vorstehende Teil der Rippenteile 17 bzw. 17a an der nächstliegenden Sollbruchstelle 41 abgebrochen. Sollte die nun entstehende Bruchstelle noch nach oben abstehen, kann das betreffende Rippenteil 17 bzw. 17a mit weiteren Schlägen weiter eingeschlagen werden, bis das obere Ende des Rippenteils bündig mit der Schulter 21 am oberen Ende des Schaftblattes 1 ist.

Zur Sicherung der Rippenteile 17 bzw. 17a in der eingeführten Lage ist, wie in Fig. 3 dargestellt ein Deckelteil 42 vorgesehen, das mittels eines Schraubbolzens 43 auf der Schulter 21 am oberen Ende des Schaftblattes 1 verankert wird. Die Schulter 21 ist, wie in Fig. 3 deutlich ersichtlich, von einer Nut 44 mit T-förmigem Profil durchquert. Vom Grund dieser Nut 44 geht eine Gewindebohrung 45 zur Aufnahme des Schraubbolzens 43 aus. Die Nut 44 dient unter anderen Dingen auch dazu, die Prothese (nachdem das Deckelteil 42 entfernt wurde) mit einer entsprechenden Handhabe (nicht dargestellt) aus der Implantationshöhlung herauszuziehen.

Die erfindungsgemässe Ausgestaltung des halsnahen Bereichs ist nicht auf die beispielhaft dargestellte Prothese mit geradem und symmetrischem Schaftblatt beschränkt. Die gezeigte Anordnung von Nuten und passenden Rippenteilen ist für jede Prothese geeignet, welche am proximalen Ende des Femurs verankert werden muss.

## Patentansprüche

1. Femurprothese mit einem Schaftblatt (1), einem zur Aufnahme einer Gelenkkugel bestimmten Hals (6), mit im halsnahen Bereich (4) auf der dorsalen und ventralen Seite des Schaftblattes (1) vorgesehenen, an ihrem proximalen Ende (19) offenen Nuten (16) und mit nach dem Einführen des Schaftblattes (1) in die Implantationshöhlung, nach Massgabe der Abmessung der Implantationshöhlung in die Nuten (16) einsetzbaren, im eingesetzten Zustand über das Schaftblatt (1) vorstehenden Rippenteilen (17), dadurch gekennzeichnet, dass zu beiden Seiten des Schaftblattes (1) mehrere in in Längsrichtung des Schaftblattes (1) verlaufende Nuten (16) angeordnet sind, die je zur Aufnahme eines eine einzige Rippe (24) bildenden Rippenteils (17) bestimmt sind, und dass Verriegelungsmittel (22, 29, 30, 42) zum Sichern der in die Nuten (16) eingesetzten Rippenteile (17) gegen eine Längsverschiebung gegenüber dem Schaftblatt (1) vorgesehen sind.

2. Femurprothese nach Anspruch 1, dadurch gekennzeichnet, dass die Nuten (16) parallel zueinander verlaufen.

3. Femurprothese nach Anspruch 1 oder 2, dadurch gekennzeichnet, dass auf der lateralen Seite (10) des Schaftblattes (1) eine weitere Nut (23) vorgesehen ist.

4. Femurprothese nach einem der Ansprüche 1 bis 3, dadurch gekennzeichnet, dass das Nutenprofil (33) sich gegen das Schaftblattinnere erweitert und dass jedes Rippenteil (17, 17a) einen Rippenfuss (25) mit gegengleichem Profil (34) aufweist.

5. Femurprothese nach Anspruch 4, dadurch gekennzeichnet, dass das Nutenprofil (33) und der Rippenfuss (25) schwalbenschwanzförmig sind.

6. Femurprothese nach Anspruch 1 oder 2, dadurch gekennzeichnet, dass zu beiden Seiten des Schaftblattes (1) eine quer zu den Nuten (16) verlaufende, diese durchsetzende, und im Nutengrund (28) eine Kerbe (28a) bildende Bohrung (22) vorgesehen ist, das im Fuss (25) der Rippenteile (17) eine Kerbe (26) vorgesehen ist und dass bei eingesetzten Rippenteilen (17) ein in die Bohrung (22) eingeschobener Stift (32a) in die Kerben (26) der Rippenteile (17) eingreift und diese verriegelt.

7. Femurprothese nach Anspruch 6, dadurch gekennzeichnet, dass die Stifte (32a) durch die Schenkel (32) eines U-förmigen Bügels (30) gebildet sind, der in eingeschobener Stellung mit seinem Steg (31) von einer Rille (29) auf der Schmalseite (9) des Schaftblattes (1) aufgenommen ist.

8. Femurprothese nach den Ansprüchen 3 und 7, dadurch gekennzeichnet, dass der Bügel (30) in eingeschobener Stellung mit seinem Steg (31) in eine Kerbe (27) des in der weiteren Nut (23) eingeschobenen Rippenelements (17a) eingreift und dieses verriegelt.

9. Femurprothese nach Anspruch 1 oder 2, dadurch gekennzeichnet, dass die Nuten (16) sich über die gesamte Länge des Schaftblattes (1) erstrecken.

10. Femurprothese nach Anspruch 9, dadurch gekennzeichnet, dass die Rippenteile (17, 17a) eine in Längsrichtung des Schaftblattes (1) abnehmende Höhe aufweisen.

11. Femurprothese nach Anspruch 10, dadurch gekennzeichnet, dass die Höhe der Rippenteile (17, 17a) stufenweise abnimmt und dass die Rippenteile (17, 17a) zwischen Abschnitten (40) konstanter Höhe durch Kerben (41) gebildete Sollbruchstellen aufweisen.

12. Femurprothese nach einem der Ansprüche 9-11, dadurch gekennzeichnet, dass die Verriegelungsmittel einen lösbar mit dem Schaftteil (1) verbundenen, das proximale Ende der Nuten (16) abschliessenden Deckel (42) aufweisen.

13. Femurprothese nach einem der Ansprüche 9 bis 12, dadurch gekennzeichnet, dass ihr ein Satz Abstandhalter (35) zugeordnet ist, von denen einer in die mediale Schmalseite (5) des Schaftblattes (1) einsetzbar ist, um die Krümmung im Uebergangsbereich zwischen Hals (6) und medialer Schmalseite (5) der Krümmung des Calcarbogens der Implantationshöhlung anzupassen.

## Claims

1. A femoral prosthesis with a shank leaf (1), a neck (6) intended to receive an articulation ball and with, in the area (4) which is close to the neck and on the dorsal and ventral sides or the shank leaf (1), grooves (16) which are open at their proximal end (19) and with fin parts (17) which, after the shank leaf (1) has been introduced into the implantation cavity, can be inserted into the grooves (16) in accordance with the dimensions of the implantation cavity and which project beyond the shank leaf (1) in the inserted state, characterised in that on both sides of the shank leaf (1) there are a plurality of grooves (16) extending in the longitudinal direction of the shank leaf (1), each groove (16) being intended to accommodate a fin part (17) forming a single rib (24), and in that locking means (22, 29, 30, 42) are provided for securing the fin parts (17) inserted into the grooves (16) to prevent their longitudinal displacement in relation to the shank leaf (1).

2. A femoral prosthesis according to claim 1, characterised in that the grooves (16) extend parallel with one another.

3. A femoral prosthesis according to Claim 1 or 2, characterised in that a further groove (23) is provided on the lateral side (10) of the shank leaf (1).

4. A femoral prosthesis according to one of Claims 1 to 3, characterised in that the groove profile (33) widens out towards the interior of the shank leaf and in that each fin part (17, 17a) has a fin root (25) of diametrically opposite profile (34).

5. A femoral prosthesis according to Claim 4, characterised in that the groove profile (33) and fin root (25) are dovetailed in shape.

6. A femoral prosthesis according to Claim 1 or 2, characterised in that on both sides of the shank leaf (1) there is extending transversely to and traversing the grooves (16) and forming in the groove bottom (28) a notch (28a), a bore (22) and in that a notch (26) is provided in the root (25) of the fin parts (17) and in that when the fin parts (17) are inserted, a peg (32a) inserted into the bore (22) engages the notches (26) in the fin parts (17) and locks them.

7. A femoral prosthesis according to Claim 6, characterised in that the pegs (32a) are formed by the arms (32) of a U-shaped member (30) which, in the pushed-in position, has its web (31) housed in a groove (29) on the narrow side (9) of the shank leaf (1).

8. A femoral prosthesis according to Claims 3 and 7, characterised in that when the member (30) is in the pushed-in position, its web (31) engages a notch (27) in the fin element (17a) which is pushed into the further groove (23) and locks the said fin element (17a).

9. A femoral prosthesis according to Claim 1 or 2, characterised in that the grooves (16) extend over the total length of the shank leaf (1).

10. A femoral prosthesis according to Claim 9, characterised in that the fin parts (17, 17a) are of a height which diminishes in the longitudinal direction of the shank leaf (1).

11. A femoral prosthesis according to Claim 10, characterised in that the height of the fin parts (17, 17a) diminishes in a step-wise manner and in that the fin parts (17, 17a) have intended fracture points consisting of notches (41) between portions (40) of constant height.

12. A femoral prosthesis according to one of Claims 9 to 11, characterised in that the locking means comprise a cap (42) closing off the proximal end of the grooves (16) and separably connected to the shank portion (1).

13. A femoral prosthesis according to one of Claims 9 to 12, characterised in that associated with it is a set of spacers (35) one of which can be inserted into the medial narrow side (5) of the shank leaf (1) in order to adapt the curvature in the transition zone between neck (6) and medial narrow side (5) to the curvature of the calcaric arch in the implantation cavity.

## Revendications

1. Prothèse fémorale, comprenant une lame de tige (1), un col (6) destiné à recevoir une rotule, une section (4) proche du col prévue sur les faces dorsale et ventrale de la lame de tige (1) des rainures (16) ouvertes à leur extrémité proximale (19) et des éléments d'ailette (17) qui, après l'introduction de la lame de tige (1) dans la concavité d'implantation, peuvent être insérés, en fonction des dimensions de la concavité d'implantation, dans les rainures (16) et dépassent, à l'état inséré, de la lame de tige (1), **caractérisée en ce** que des deux côtés de la lame de tige (1) sont disposées plusieurs rainures (16) qui s'étendent dans la direction longitudinale de la lame de tige (1) et servent à chaque fois à recevoir un élément d'ailette (17) formant une seule ailette (24) et que des moyens de verrouillage (22, 29, 30, 42) sont prévus pour empêcher un déplacement longitudinal des éléments d'ailette (17) insérés dans les rainures (16) par rapport à la lame de tige (1).

2. Prothèse fémorale selon la revendication 1, caractérisée en ce que les rainures (16) sont parallèles.

3. Prothèse fémorale selon l'une des revendications 1 ou 2, caractérisée en ce que sur la face latérale (10) de la lame de tige (1) est prévue une rainure supplémentaire (23).

4. Prothèse fémorale selon l'une des revendications 1 à 3, caractérisée en ce que le profil (33) des rainures s'élargit vers l'intérieur de la lame de tige et que chaque élément d'ailette (17, 17a) comporte un pied d'ailette (25) avec un profil conjugué (34).

5. Prothèse fémorale selon la revendication 4, caractérisée en ce que le profil (33) des rainures et le pied (25) des ailettes sont conformés en queue d'aronde.

6. Prothèse fémorale selon l'une des revendications 1 ou 2, caractérisée en ce que des deux côtés de la lame de tige (1) est prévu un alésage (22) qui est orienté transversalement aux rainures (16), les traverse et forme une encoche (28a) au fond (28) desdites rainures, que dans le pied (25) des éléments d'ailette (17) est prévue une encoche (26), et que, lorsque les éléments d'ailette (17) sont insérés, une broche (32a) introduite dans l'alésage (22) s'engage dans les encoches (26) des éléments d'ailette (17) et verrouille ces derniers.

7. Prothèse fémorale selon la revendication 6, caractérisée en ce que les broches (32a) sont constituées par les branches (32) d'un étrier (30) en forme de U dont le dos (31) est logé, dans la position engagée, dans une gorge (29) sur la face étroite (9) de la lame de tige (1).

8. Prothèse fémorale selon l'une des revendications 3 et 7, caractérisée en ce que, dans la position engagée, le dos (31) de l'étrier (30) s'engage dans une encoche (27) de l'élément d'ailette (17a) inséré dans la rainure supplémentaire (23) en verrouillant ledit élément d'ailette.

9. Prothèse fémorale selon l'une des revendications 1 ou 2, caractérisée en ce que les rainures (16) s'étendent sur toute la longueur de la lame de tige (1).

10. Prothèse fémorale selon la revendication 9, caractérisée en ce que les éléments d'ailette (17, 17a) présentent une hauteur qui diminue dans la direction longitudinale de la lame de tige (1).

11. Prothèse fémorale selon la revendication 10, caractérisée en ce que la hauteur des éléments d'ailette (17, 17a) diminue par étapes, et que les éléments d'ailette (17, 17a) comportent des zones de rupture imposée formées par des encoches (41) entre des sections (40) de hauteur constante.

12. Prothèse fémorale selon l'une des revendications 9 à 11, caractérisée en ce que les moyens de verrouillage comprennent un couvercle (42) relié de manière amovible à l'élément de tige (1) et obturant l'extrémité ouverte des rainures (16).

13. Prothèse fémorale selon l'une des revendications 9 à 12, caractérisée en ce qu'il lui est associé un jeu d'écarteurs (35) dont l'un peut être inséré dans la face étroite médiane (5) de la lame de tige (1) pour adapter la courbure dans la zone de transition entre le col (6) et la face étroite médiane (5) à la courbure de l'arc du calcar fémoral de la concavité d'implantation.
